(19) Europäisches Patentamt
European Patent Office
Office européen des brevets

(11) **EP 3 585 982 B1**

(12) **FASCICULE DE BREVET EUROPEEN**

(45) Date de publication et mention de la délivrance du brevet:
**21.04.2021 Bulletin 2021/16**

(21) Numéro de dépôt: **18710097.9**

(22) Date de dépôt: **21.02.2018**

(51) Int Cl.:
*F01D 21/00* $^{(2006.01)}$    *G01N 21/25* $^{(2006.01)}$

(86) Numéro de dépôt international:
**PCT/FR2018/050407**

(87) Numéro de publication internationale:
**WO 2018/154235 (30.08.2018 Gazette 2018/35)**

(54) **PROCEDE DE CONTROLE NON DESTRUCTIF D'UN CARTER PAR COLORIMETRIE**

VERFAHREN ZUR ZERSTÖRUNGSFREIEN PRÜFUNG EINES GEHÄUSES DURCH KOLORIMETRIE

METHOD FOR NON-DESTRUCTIVE TESTING OF A CASING BY COLORIMETRY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.02.2017 US 201762462154 P**
**08.03.2017 FR 1751896**

(43) Date de publication de la demande:
**01.01.2020 Bulletin 2020/01**

(73) Titulaire: **Safran Aircraft Engines**
**75015 Paris (FR)**

(72) Inventeurs:
• **ROMERO, Jean-Louis**
**77550 Moissy-Cramayel (FR)**
• **COULETTE, Jean-Pierre**
**77550 Moissy-Cramayel (FR)**
• **MAINCZYK, Angélique, Melody, Marine, Alexia**
**77550 Moissy-Cramayel (FR)**

(74) Mandataire: **Ernest Gutmann - Yves Plasseraud S.A.S.**
**66, rue de la Chaussée d'Antin**
**75009 Paris (FR)**

(56) Documents cités:
**WO-A1-2013/050691     JP-A- S6 159 242**
**JP-A- 2008 180 607     US-A1- 2005 067 569**
**US-A1- 2014 096 350**

EP 3 585 982 B1

## Description

La présente invention concerne un procédé de contrôle non destructif d'une pièce en matériau polymère. En particulier, des fibres de renfort peuvent être intégrées à la matrice.

Classiquement, l'extrémité amont d'une turbomachine comprend une soufflante comprenant une roue formée d'une pluralité d'aubes entourées extérieurement par un carter annulaire qui peut être réalisé en matériau métallique ou en matériau composite comprenant une matrice intégrant des fibres de renfort, telle qu'une matrice polymère par exemple du polymère époxyde, et des fibres de renforts en fibre de carbone ou fibres de verre. Ce carter permet une compression initiale de l'air entrant dans la turbomachine et assure également une fonction de confinement des aubes en cas de perte de l'une d'entre elles. Le carter de soufflante est entouré par une pluralité de conduits d'alimentation d'équipements, notamment par un conduit d'alimentation en air sous pression, à une température de l'ordre de 200°C, d'un moteur nommé APU (auxiliary power unit) servant au démarrage du turboréacteur ainsi qu'à l'alimentation en électricité de la cabine de l'avion lorsque l'avion est au sol.

En cas de disfonctionnement, telle qu'une fuite, de la conduite d'alimentation en air sous pression, l'air peut conduire à un échauffement local important du carter puisque la température de l'air est de l'ordre de 200°C. Lorsque le carter est réalisé en matériau métallique, par exemple en aluminium, l'échauffement n'impacte par l'intégrité mécanique du carter. Dans le cas d'un carter à matrice intégrant des fibres de renforts, sa tenue mécanique suite à un échauffement doit pouvoir être garantie.

On comprend donc que le contrôle non destructif d'un carter composite à matrice avec fibres de renforts est particulièrement important et l'est d'autant plus qu'un carter composite s'avère très couteux.

Il a ainsi été proposé d'appliquer des peintures thermosensibles sur le carter. Toutefois, la durée de vie de ces peintures limite fortement leur intérêt puisqu'un moteur peut être utilisé durant des périodes supérieures à la durée de vie de ces peintures, en particulier pour les avions du type longs ou moyens courriers. En outre, lors de la dépose d'un moteur, celui-ci subit de manière conventionnelle un nettoyage par décapage qui induit un retrait total de la couche de peinture thermosensible, impliquant une nouvelle étape d'application d'une couche de peinture. Enfin, si une peinture thermosensible permet de rendre visuellement compte de l'état de chauffe d'une zone donnée d'un carter, elle ne s'avère être qu'une mesure indirecte de l'état de la structure interne du carter et ne permet pas une quantification précise de la structure interne du carter. WO 2013/050691 divulgue un procédé de contrôle non destructif de l'échauffement d'une zone déterminée à contrôler d'une pièce en matériau polymère.

L'invention a notamment pour but d'apporter une solution simple, efficace et économique aux problèmes de l'art antérieur décrit précédemment.

A cet effet, elle propose un procédé de contrôle non destructif de l'échauffement d'une zone déterminée à contrôler d'une pièce en matériau polymère, le procédé comportant les étapes suivantes :

a) réaliser au moins une mesure de colorimétrie sur ladite zone déterminée à contrôler et obtenir la valeur $a_p$ du paramètre $a$ de l'espace colorimétrique CIELAB,

b) réaliser au moins une mesure de colorimétrie sur une zone de référence de ladite pièce et obtenir la valeur $a_{p/ref}$ du paramètre a de l'espace colorimétrique CIELAB,

c) calculer $\Delta a_p = a_p - a_{p/ref}$,

d) établir un risque d'échauffement de ladite zone déterminée à contrôler si $\Delta a_p$ est supérieur à une valeur seuil **A1**.

Le procédé selon l'invention permet de déterminer si la pièce examinée a subi ou non une surchauffe au-delà d'une limite acceptable. En pratique, si un risque de surchauffe est établi, une analyse physico-chimique de la pièce est réalisée de manière à déterminer plus précisément le niveau d'échauffement. Toutefois, une analyse physico-chimique doit être effectuée en laboratoire, ce qui nécessite un démontage de la pièce de l'ensemble mécanique sur lequel elle est montée. Ainsi, lorsque la pièce à contrôler est un carter de la turbomachine, le contrôle en laboratoire impose une dépose du moteur de l'avion et augmente fortement les temps d'immobilisation de l'avion et par conséquent les coûts d'exploitation.

L'invention propose d'établir un risque d'échauffement d'une zone donnée d'une pièce par comparaison à une partie de référence supposée saine, c'est-à-dire n'ayant pas subi d'échauffement, de la pièce examinée, ce qui permet d'établir une référence sur la pièce elle-même afin de tenir compte des variations normales du paramètre a dues à l'environnement externe dans lequel la pièce a vécu depuis sa fabrication. Le terme « normale » se réfère aux conditions standards d'utilisation de la pièce permettant d'avoir une durée de vie prédéterminée de la pièce.

Dans l'espace colorimétrique CIELAB, la variation de la valeur du paramètre a renseigne sur le niveau de surchauffe subi par la zone déterminée à contrôler de la pièce.

Le seuil A1 peut être déterminé en utilisant une base de données de référence comprenant des mesures de colorimétrie réalisées sur une pluralité d'échantillons de référence en matériau polymère, en particulier à fibres de renfort, ayant été soumis à une température déterminée durant une période de temps déterminée. A partir d'une pluralité de mesures du paramètre a, on établit un seuil A1 en-deçà duquel l'intégrité mécanique de la pièce ne peut pas être garantie sans analyse physico-chimique.

La valeur $\Delta a_p$ renseigne sur le niveau (composante) de couleur rouge présent dans la zone donnée qui est contrôlée.

Dans la demande, l'espace colorimétrique CIELAB désigne le système CIE$L*a*b*$ ou l'acronyme CIE désigne la Commission Internationale de l'Eclairage. Les astérisques ont été volontairement omis dans le texte pour éviter d'alourdir les notations.

Dans le présent document l'espace CIELAB correspond à celui défini par la norme NF EN ISO 11664-4 (2011-07-01) dont le titre est « Colorimétrie - Partie 4 : espace chromatique L*a*b* CIE 1976 ».

Le procédé peut en outre comprendre les étapes suivantes :

- obtenir la valeur $b_p$ du paramètre b et la valeur $L_p$ du paramètre $L$ de ladite au moins une mesure de colorimétrie effectuée à l'étape a),
- obtenir la valeur $b_{p/ref}$ du paramètre b et la valeur $L_{p/ref}$ du paramètre $L$ de ladite au moins une mesure de colorimétrie effectuée à l'étape b),
- calculer $\Delta b_p = b_p - b_{p/ref}$ et calculer $\Delta L_p = L_p - L_{P/ref}$,
- établir un risque d'échauffement de ladite zone déterminée à contrôler si toutes les conditions suivantes sont vérifiées :

  ◦ $\Delta a_p$ est supérieur à une valeur seuil $A2$, $A2$ étant inférieur à $A1$,
  ◦ $\Delta b_p$ est supérieur à une valeur seuil $B1$,
  ◦ $\Delta L_p$ est inférieur à une valeur seuil $L1$.

La valeur $\Delta b_p$ renseigne sur le niveau de jaunissement de la zone déterminée qui est contrôlée. Il est rappelé que, dans cet espace de couleurs, la valeur du paramètre $b$ évolue depuis des valeurs négatives jusqu'à des valeurs positives, c'est-à-dire du bleu vers le jaune. La variation de la valeur du paramètre $L$ renseigne sur l'évolution de la luminance/clarté et évolue de la valeur 0 correspondant au noir jusqu'à la valeur 100 correspond au blanc. Le seuil $L1$ permet de renseigner sur la diminution de clarté de la zone contrôlée par rapport à la zone de référence.

Ainsi, si lors de l'étape d), le risque de surchauffe n'est pas établi, le procédé consiste à effectuer trois comparaisons successives dont les résultats positifs impliquent l'établissement d'un risque.

Selon une caractéristique de l'invention, les seuils $A2$, $B1$, $L1$ sont déterminés en utilisant lesdites mesures de colorimétrie stockées dans la base de données de référence.

Selon une autre caractéristique de l'invention, l'établissement de la base de données comprend les étapes suivantes :

- pour chaque premier échantillon, obtenir la valeur $a'$ du paramètre a de l'espace colorimétrique CIELAB, à partir d'au moins une mesure de colorimétrie,
- pour chaque premier échantillon, calculer $\Delta a' = a' - $

$a'_{ref}$, où :
◦ $a'_{ref}$ correspond à la valeur du paramètre $a$ de l'espace colorimétrique CIELAB, cette valeur ayant été obtenue sur un second échantillon en matériau polymère, en particulier à fibres de renfort, ayant le même temps d'existence que ledit premier échantillon considéré et ayant été maintenu à une température comprise dans une plage de températures, telle que celle de préservation de l'intégrité mécanique du second échantillon ou bien telle qu'une plage entre 0 et 40 °C pouvant prendre en compte en outre l'exposition aux rayonnements dans le domaine ultraviolet,

- effectuer un test de détermination des propriétés mécaniques de chacun des premiers échantillons,
- déterminer le seuil $A1$ à partir d'une comparaison des données issues des tests effectués à l'étape précédente et des valeurs $\Delta a'$ contenues en base de données.

L'établissement de la base de données comprend également les étapes suivantes :

- pour chaque premier échantillon, obtenir les valeurs $L'$ et $b'$ des paramètres respectifs $L$ et $b$ de l'espace colorimétrique CIELAB, à partir d'au moins une mesure de colorimétrie,
- pour chaque premier échantillon, calculer $\Delta b' = b' - b'_{ref}$ et $\Delta L' = L' - L'_{ref}$, où :
  ◦ $b'_{ref}$ et $L'_{ref}$ correspondent respectivement aux valeurs des paramètres $b$ et $L$ de l'espace colorimétrique CIELAB, ces valeurs ayant été obtenues sur un second échantillon en matériau polymère, en particulier à fibres de renfort, ayant le même temps d'existence que ledit premier échantillon considéré et ayant été maintenu à une température comprise dans une plage de températures, telle que celle de préservation de l'intégrité mécanique du second échantillon ou bien telle qu'une plage entre 0 et 40 °C pouvant prendre en compte en outre l'exposition aux rayonnements dans le domaine ultraviolet,
- déterminer les seuils $A2$, $B1$ et $L1$ à partir d'une comparaison des données issues desdits tests et des valeurs $\Delta a'$, $\Delta b'$ et $\Delta L'$ contenues en base de données.

Selon l'invention, le test des propriétés mécaniques des premiers échantillons peut comprendre au moins une étape de test mécanique de la pièce, par exemple en traction et/ou compression.

Le procédé peut encore comprendre les étapes suivantes :

- calculer la différence de couleur $\Delta E_p$ entre les mesures de colorimétrie obtenues aux étapes a) et b),
- à partir de la valeur $\Delta E_p$ obtenue à l'étape précédente, déterminer la période de temps durant laquelle ladite zone à contrôler de la pièce a été soumise à une température d'échauffement déterminée et déterminer ladite température d'échauffement, en utilisant une base de donnée de référence comprenant des valeurs $\Delta E'$ de différence de couleurs obtenues à partir d'une pluralité d'échantillons en matériau polymère ayant été soumis à une température déterminée durant une période de temps déterminée.

La valeur $\Delta E_p$ est déterminée pour ladite zone à contrôler en effectuant le calcul

$$\Delta E_p = \sqrt{\Delta L_p{}^2 + \Delta a_p{}^2 + \Delta b_p{}^2}.$$

Les valeurs $\Delta E'$ sont déterminées pour chaque premier échantillon en effectuant le calcul

$$\Delta E' = \sqrt{\Delta L'^2 + \Delta a'^2 + \Delta b'^2}.$$

Pour limiter les erreurs de mesure et moyenner la variabilité expérimentale, chaque valeur considérée des paramètres $L, a, b$ de l'espace colorimétrique CIELAB peut être obtenue en effectuant la moyenne d'au moins cinq mesures successives de colorimétrie à l'endroit considéré.

Selon l'invention, en cas d'établissement d'un risque d'échauffement, le procédé peut également comprendre en outre une étape subséquente à l'étape d) de réalisation d'une analyse physico-chimique de la zone déterminée à contrôler de la pièce de manière à statuer sur l'état d'endommagement thermique de ladite zone déterminée.

La pièce analysée peut être en matériau polymère comprenant des fibres de renfort.

De préférence, une étape de nettoyage de la surface sur laquelle une mesure de colorimétrie est destinée à être réalisée, est effectuée en préalable à ladite mesure de colorimétrie.

L'invention sera mieux comprise et d'autres détails, avantages et caractéristiques de l'invention apparaîtront à la lecture de la description suivante faite à titre d'exemple non limitatif, en référence aux figures suivantes :

- la figure 1 est une vue schématique d'une carter de turbomachine à contrôler ;
- la figure 2 est une vue représentation des axes de l'espace colorimétrique CIELAB utilisé dans le présent document ;
- la figure 3 est une vue schématique d'une pluralité d'échantillons ayant chacun subi une oxydation durant un temps donné (verticalement) à une température donné (horizontalement) ;
- la figure 4 est une vue schématique illustrant une ligne de séparation entre une première zone 1 et une seconde zone 2 ;
- la figure 5 est une vue schématique d'un graphe où chaque point représente un échantillon de la figure 3 ayant été soumis à une température inférieure à la température de transition vitreuse du matériau contrôlé, chaque point étant placé sur le graphe en fonction des valeurs de ses paramètres $a$ et $b$, $a$ étant représenté sur l'axe des abscisses et b étant représenté sur l'axe des ordonnées ;
- la figure 6 est une vue schématique d'un graphe où chaque point représente un échantillon de la figure 3 ayant été soumis à une température supérieure à la température de transition vitreuse du matériau polymère, chaque point étant placé sur le graphe en fonction des valeurs de ses paramètres $a$ et $b$, $a$ étant représenté sur l'axe des abscisses et $b$ étant représenté sur l'axe des ordonnées ;
- la figure 7 est un logigramme de fonctionnement du procédé de prise de décision lors d'une étape de contrôle non destructif sur une zone donnée à contrôler d'une pièce déterminée telle que le carter de la figure 1 ;
- la figure 8 est un graphe représentant l'évolution de la différence de couleur en fonction du temps pour les échantillons de la figure 3;
- la figure 9 est un graphe à plus grande échelle d'une zone donnée du graphe de la figure 8.

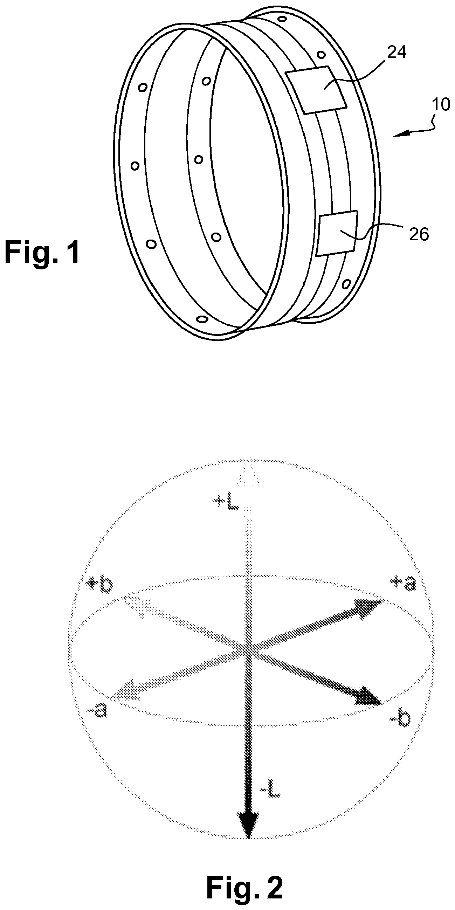

Comme expliqué précédemment, le carter 10 de soufflante représenté en figure 1, réalisé en polymère, en particulier à fibres de renfort, peut subir en fonctionnement un échauffement local qu'il convient de pouvoir caractériser par une méthode non destructive permettant de déterminer l'état du carter 10 afin de déterminer s'il peut ou non être maintenu en service dans une turbomachine.

La figure 2 représente l'espace CIELAB utilisé dans le présent document pour effectuer l'analyse des données colorimétriques obtenues sur la pièce à contrôler ainsi que pour établir la base de données de référence sur une pluralité de premiers échantillons. L'espace CIELAB est un système qui permet de représenter les composantes trichromatiques suivant trois axes orthogonaux entre eux. L'axe $L$ (ou $L^*$) représente l'axe de luminosité ou de clarté (luminance) (noir parfait : $L = 0$ ; blanc parfait : $L = 100$). L'axe $a$ (ou $a^*$) représente l'axe allant du vert (valeurs négatives de $a$) au rouge (valeurs positives de $a$). L'axe b (ou $b^*$) représente l'axe allant du bleu (valeurs négatives de b) au jaune (valeurs positives de b).

Il est de nouveau rappelé que l'espace utilisé est l'espace colorimétrique CIE $L^*a^*b^*$ et que les astérisques ont été volontairement supprimés comme cela est usuel.

Dans ce système de couleur, une différence de couleurs entre une première couleur de coordonnées $L_1, a_1, b_1$ et une seconde couleur de coordonnées $L_2, a_2, b_2$ est calculée comme suit :

$$\Delta E = \sqrt{\Delta L^2 + \Delta a^2 + \Delta b^2},$$

où

$$\Delta L = (L_1 - L_2)$$

$$\Delta a = (a_1 - a_2)$$

$$\Delta b = (b_1 - b_2)$$

Ce mode de calcul de la différence de couleurs est celui utilisé ultérieurement comme cela apparaitra dans la suite de la description.

L'invention propose d'établir une base de données de référence comprenant des mesures colorimétriques selon le système de couleur CIELAB. Le terme « référence » utilisé ci-après est à comprendre comme désignant un élément de la base de données de référence comprenant les mesures de colorimétrie et plus généralement les données obtenues à partir des échantillons de référence.

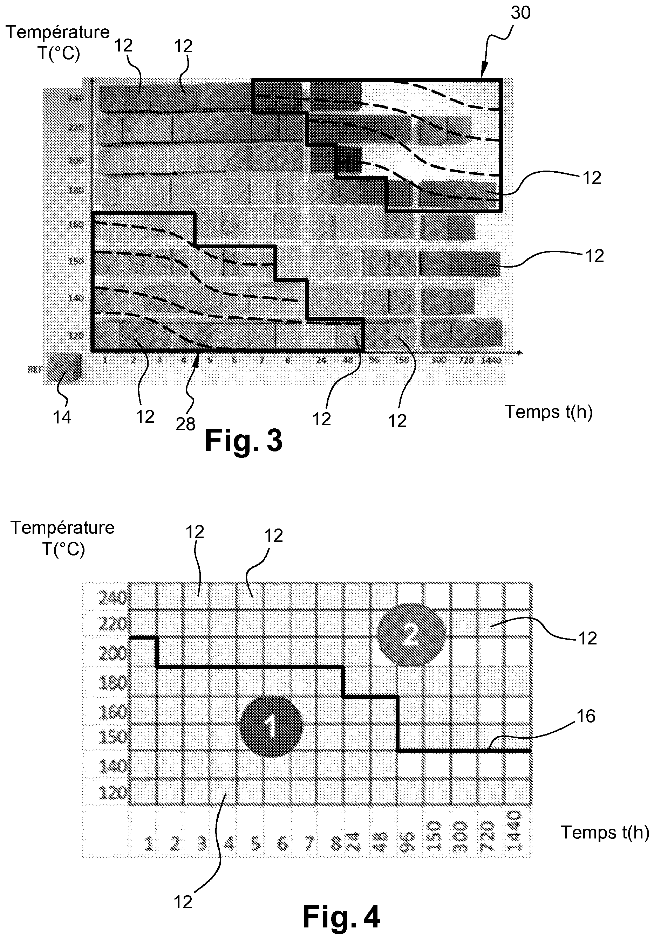

Pour cela, un lot d'une pluralité de premiers échantillons 12 d'un matériau similaire à la pièce à analyser est constitué. La figure 3 illustre un tel lot qui comprend ainsi plusieurs premiers échantillons 12 de carter 10 en matériau polymère, de préférence à fibres de renfort, agencés sous forme de lignes et de colonnes. Le long d'une ligne donnée, chaque premier échantillon 12 est soumis à une température donnée dont le temps d'exposition est donné par la position le long d'une ligne. Bien évidemment, la base de données devrait comprendre un nombre de premiers échantillons 12 permettant de rendre compte des différents niveaux d'oxydation thermique que le polymère peut subir en conditions réelles de fonctionnement. Ainsi, la base de données devrait comprendre des premiers échantillons 12 ayant été soumis aux températures précitées durant des périodes de temps allant au moins jusqu'à 6 mois.

Dans la configuration représentée à titre d'exemple, les premiers échantillons 12 ont été soumis à des températures en °C de 120, 140, 150, 160, 180, 200, 220 et 240°C durant des périodes en heures s'étalant de 1 jusqu'à 1440 heures qui correspond à une période de 2 mois. L'échantillon 14 positionné dans le coin inférieur gauche de la figure 1 représente un échantillon 12 n'ayant subi aucun échauffement, lequel constitue donc la référence absolu d'un carter 10 sans aucun échauffement thermique. On constate sur la figure 1 que les premiers échantillons 12 s'assombrissent au fur et à mesure que la température augmente et que le temps d'exposition à une température augmente, ce qui est cohérent avec une oxydation thermique du polymère.

La figure 4 représente de manière schématique la figure 3 et comprend une ligne de séparation 16 d'une première zone 1 et d'une seconde zone 2. La première zone 1 correspond à des premiers échantillons 12 qui ont été soumis à une température acceptable durant un période de temps acceptable alors que la seconde zone 2 correspond à des premiers échantillons 12 qui ont été soumis à une température trop importante durant une période de temps donnée. Ainsi, si visuellement il est possible d'établir cette ligne de séparation, il apparait nécessaire d'établir un ou plusieurs paramètres permettant de rendre compte de manière objective de l'état d'une pièce analysée. C'est ce qui est décrit ci-après par l'établissement de la base de données de référence.

L'établissement de la base de données de référence consiste en premier lieu à effectuer une mesure de colorimétrie pour chacun des premiers échantillons et en déduire les valeurs L', a' et b' des paramètres L, a, b de l'espace colorimétrique CIELAB, à partir d'au moins une mesure de colorimétrie.

Notons tout de suite que les valeurs des paramètres L, a, b peuvent être obtenus à partir de plusieurs mesures de colorimétrie dans chaque zone où une mesure est effectuée, c'est-à-dire pour établir la base de données ou alors lorsque l'on souhaite contrôler une zone déterminée d'une pièce comme cela sera expliqué ultérieurement.

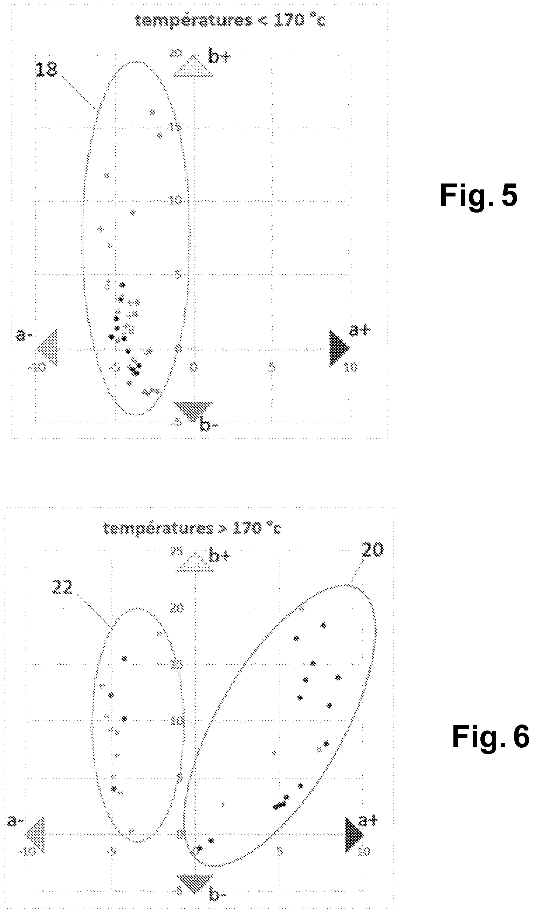

Il est maintenant fait référence aux figures 5 et 6 représentant chacune un graphe où chaque point représente un premier échantillon de la figure 3, chaque point étant placé sur le graphe en fonction des valeurs de ses paramètres $a$ et $b$, $a$ étant représenté sur l'axe des abscisses et b étant représenté sur l'axe des ordonnées. Sur la figure 5, les premiers échantillons ont été soumis à une température inférieure à la température de transition vitreuse du matériau polymère, ici 170°C, et sur la figure 6, les premiers échantillons ont été soumis à une température supérieure à ladite température de transition vitreuse.

Sur la figure 5, on constate qu'un groupe 18 de points se trouve à des valeurs du paramètre a inférieure à zéro alors que, sur la figure 6, pour les premiers échantillons ayant été soumis à une température supérieure à 170°C, on constate qu'un groupe 20 de valeurs du paramètre a sont supérieures à zéro. Dès lors, il est possible d'effectuer une discrimination sur l'état thermique, c'est à dire d'échauffement d'une zone donnée d'une pièce à partir de la mesure de ce paramètre. On notera que sur la figure 6, un second groupe 22 a des valeurs du paramètre a qui sont inférieures à zéro mais ces points correspondent à des temps d'exposition très faible et inférieur à 10h qui ne sont pas à prendre en compte. Sur le graphe de la figure 5, on observe également que la variation s'effectue principalement le long de l'axe $b$, cette variation permettant de mettre en évidence le jaunissement par vieillissement naturel au cours du temps du polymère et dans le cas d'un carter de soufflante de la résine. Cette variation selon l'axe $b$ est également visible sur la figure 6.

Ainsi, on comprend qu'il est possible, avec une mesure de colorimétrie dans l'espace CIELAB, de faire la différence entre le vieillissement naturel du carter et une surchauffe accidentelle en effectuant une comparaison avec une base de données de référence.

Pour chaque premier échantillon 12, on calcule $\Delta a' = a' - a'_{ref}$, $\Delta b' = b' - b'_{ref}$ et $\Delta L' = L' - L'_{ref}$, où :

- $a'_{ref}$, $b'_{ref}$ et $L'_{ref}$ correspondent respectivement aux valeurs des paramètres $a$, $b$, $L$ de l'espace colorimétrique CIELAB, ces valeurs ayant été obtenues sur un second échantillon 14 en matériau polymère, en particulier à fibres de renfort, ayant le même temps d'existence que ledit premier échantillon considéré et ayant été maintenu à une température comprise dans une plage de températures, telle que celle de préservation de l'intégrité mécanique du second échantillon 14 ou bien telle qu'une plage entre 0 et 40 °C pouvant prendre en compte en outre l'exposition aux rayonnements dans le domaine ultraviolet.

La mesure colorimétrique sur le second échantillon 14 associé à chaque mesure d'un premier échantillon 12 peut être effectuée avec l'échantillon de référence 14 que l'on a conservé dans les conditions évoquées au paragraphe précédent.

Pour chaque premier échantillon 12, on effectue ensuite un test visant à déterminer ses propriétés mécaniques afin de statuer sur son aptitude ou non à constituer un échantillon utilisable dans des conditions déterminées. Ainsi, on détermine si l'échauffement subi par chaque premier échantillon rend celui-ci utilisable ou non. Le test effectué peut comprendre au moins une étape de test mécanique de la pièce, par exemple en traction et/ou compression.

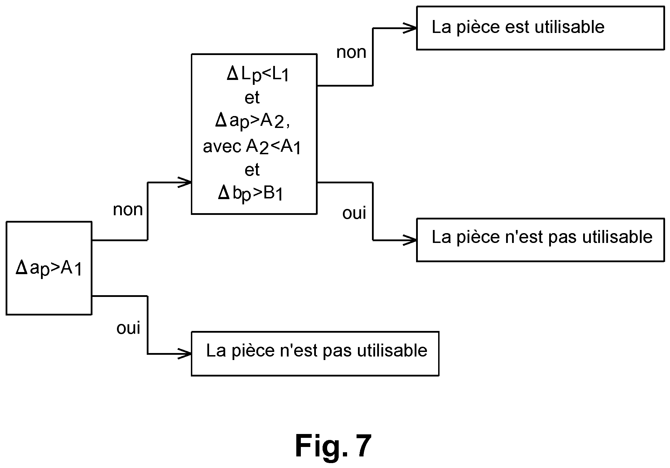

On effectue enfin une comparaison des données issues des tests avec les valeurs $\Delta a'$, $\Delta b'$ et $\Delta L'$ contenues dans la base de données de référence afin d'établir des seuils $A1$, $A2$, $B1$ et $L1$. Le seuil $A1$ correspond à un seuil au-delà duquel on considère que le carter 10 doit être déposé pour subir une inspection plus approfondie de la zone contrôler (figure 7).

Pour effectuer une opération de contrôle non destructif sur le carter 10 de la figure 1, on effectue tout d'abord les étapes suivantes, schématisées en figure 7 :

a) réalisation d'au moins une mesure de colorimétrie sur ladite zone déterminée 24 à contrôler du carter 10 et obtention de la valeur $a_p$ du paramètre a de l'espace colorimétrique CIELAB,
b) réalisation d'au moins une mesure de colorimétrie sur une zone de référence 26 du carter 10 et obtenir la valeur $a_{p/ref}$ du paramètre a de l'espace colorimétrique CIELAB,
c) calculer $\Delta a_p = a_p - a_{p/ref}$,
d) établir un risque d'échauffement de ladite zone déterminée à contrôler si $\Delta a_p$ est supérieur à une valeur seuil $A1$.

La zone de référence 26 du carter 10 est une zone qui n'a pas subi de dommage thermique.

Dans le cas où la valeur $\Delta a_p$ est inférieure, on effectue une seconde étape visant à déterminer si la pièce doit tout de même subir ou non un contrôle en prenant cette fois en compte la valeur $b_p$ du paramètre b et la valeur $L_p$ du paramètre $L$ obtenues à partir de la mesure de colorimétrie sur la zone 24 à contrôler du carter, ainsi que la valeur $b_{p/ref}$ du paramètre b et la valeur $L_{p/ref}$ du paramètre $L$ obtenues à partir de la mesure de colorimétrie sur la zone 26 de référence du carter.

Le procédé consiste ensuite à calculer $\Delta b_p = b_p - b_{p/ref}$ et calculer $\Delta L_p = L_p - L_{P/ref}$, et établir un risque d'échauffement de ladite zone déterminée 24 à contrôler si toutes les conditions suivantes sont vérifiées :

○ $\Delta a_p$ est supérieur à une valeur seuil $A2$, $A2$ étant inférieur à $A1$,
○ $\Delta b_p$ est supérieur à une valeur seuil $B1$,
○ $\Delta L_p$ est inférieur à une valeur seuil $L1$.

Dans ce cas, on cherche à déterminer si la zone testée 24 présente un jaunissement supérieur à la valeur seuil $B1$, si la clarté $\Delta L_p$ est faible, c'est-à-dire inférieur au seuil $L1$ tout en ayant un $\Delta a_p$ supérieur à une valeur seuil $A2$ inférieur à $A1$.

Si l'une des conditions précitées n'est pas vérifiée, la zone 24 à contrôler est considérée comme n'étant pas endommagée et le carter 10 peut être utilisée.

L'opération de contrôle non destructif par colorimétrie peut être effectuée sous l'aile de l'avion, ce qui permet d'avoir une décision rapide et fiable quant à la dépose ou non du carter et réduit les opérations de maintenance inutiles.

Les paramètres $A1$, $A2$, $B1$, $L1$ doivent être établis pour chaque type de pièce 10 et sont donc liés au matériau de ladite pièce et sont également fonction de l'appareil de mesure de la colorimétrie.

Ainsi, dans un exemple de réalisation des mesures colorimétriques effectuées avec un colorimètre Konica Minolta CM700d, $A1$ est égal à 4,3, $A2$ est égal à -1, $B1$ est égal à 12,6 et $L1$ est égal à -0,9.

A partir des mesures précitées de colorimétries contenues en bases de données, il est possible de déterminer la période de temps durant laquelle la zone contrôlée a été soumise à une température déterminée ainsi que cette température.

Pour cela, on calcule la différence de couleur

$$\Delta E_p = \sqrt{\Delta L_p^2 + \Delta a_p^2 + \Delta b_p^2}$$ pour la zone testée en utilisant les mesures de colorimétrie obtenues sur la zone testée et sur la zone de référence du carter analysé. Pour chacun des premiers échantillons, on calcul également la différence de couleur

$$\Delta E' = \sqrt{\Delta L'^2 + \Delta a'^2 + \Delta b'^2}$$ à partir des valeurs

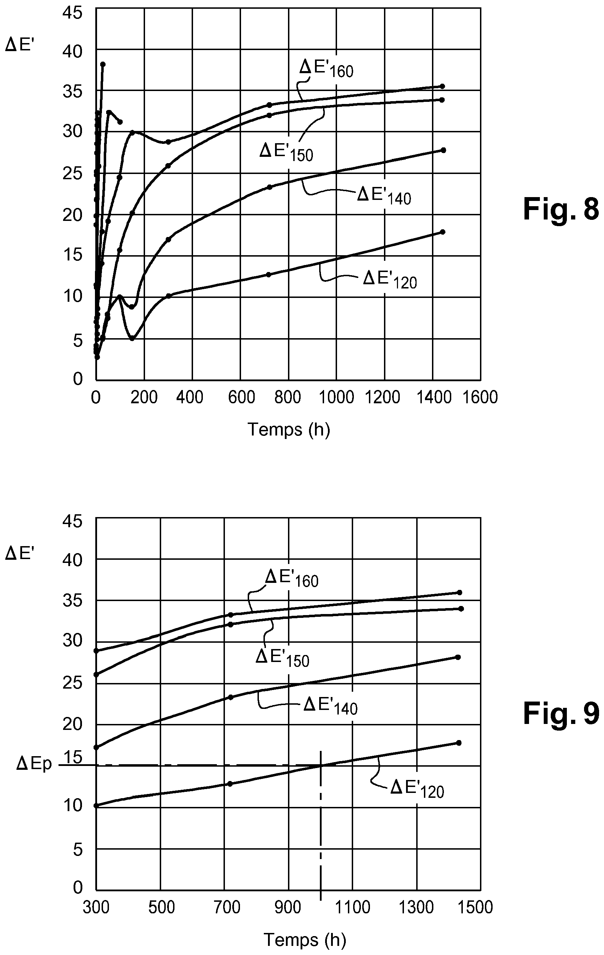

$\Delta L'$, $\Delta a'$ et $\Delta b'$. A partir des valeurs $\Delta E'$, il est possible de tracer l'évolution de $\Delta E'$ en fonction du temps pour plusieurs températures déterminées comme cela est représenté sur les figure 8 et 9.

La figure 8 représente l'évolution de $\Delta E'$ en

fonction du temps pour les températures 120, 140, 150 et 160°C, ces courbes sont respectivement notées $\Delta E'_{120}$, $\Delta E'_{140}$, $\Delta E'_{150}$ et $\Delta E'_{160}$.

Pour éviter que les courbes de l'évolution de la différence de couleurs ne soient impactées par les valeurs de $\Delta E'$ non pertinentes du fait d'une coloration très claire ou d'une coloration trop sombre, les points associés à de telles valeurs $\Delta E'$ sont supprimés. Une coloration très claire peut être due à une exposition à une température faible durant une période de temps relativement faible. Cette zone correspond à la zone 28 sur la figure 3 et ne peut pas valablement être prise en considération dans l'analyse par colorimétrie. Une coloration trop sombre peut être due à une exposition à une température trop importante durant une période de temps importante. Cette zone correspond à la zone 30 sur la figure 3 et ne peut pas valablement être prise en considération dans l'analyse par colorimétrie.

On se limite donc aux temps d'exposition relativement longs et supérieur à 300h comme cela est représenté sur la figure 9. Avec ce graphe, il est possible à partir d'une mesure $\Delta E_p$ obtenue sur une zone donnée d'un carter, de déterminer la température d'exposition en traçant la droite d'ordonnée constante $\Delta E_p$ et de rechercher la courbe interceptée qui indique donc la température subie par ladite zone contrôlée et le temps d'exposition représenté par l'abscisse du point d'intersection.

**Revendications**

1. Procédé de contrôle non destructif de l'échauffement d'une zone déterminée (24) à contrôler d'une pièce (10) en matériau polymère, le procédé comportant les étapes suivantes :

   a) réaliser au moins une mesure de colorimétrie sur ladite zone déterminée (24) à contrôler et obtenir la valeur $a_p$ du paramètre $a$ de l'espace colorimétrique CIELAB,
   b) réaliser au moins une mesure de colorimétrie sur une zone de référence (26) de ladite pièce (10) et obtenir la valeur $a_{p/ref}$ du paramètre $a$ de l'espace colorimétrique CIELAB,
   c) calculer $\Delta a_p = a_p - a_{p/ref}$,
   d) établir un risque d'échauffement de ladite zone déterminée (24) à contrôler si $\Delta a_p$ est supérieur à une valeur seuil $A1$.

2. Procédé selon la revendication 1, comprenant en outre les étapes suivantes :

   - obtenir la valeur $b_p$ du paramètre b et la valeur $L_p$ du paramètre $L$ de ladite au moins une mesure de colorimétrie effectuée à l'étape a),
   - obtenir la valeur $b_{p/ref}$ du paramètre b et la valeur $L_{p/ref}$ du paramètre $L$ de ladite au moins une mesure de colorimétrie effectuée à l'étape b),
   - calculer $\Delta b_p = b_p - b_{p/ref}$ et calculer $\Delta L_p = L_p - L_{P/ref}$,
   - établir un risque d'échauffement de ladite zone déterminée (24) à contrôler si toutes les conditions suivantes sont vérifiées :

     ◦ $\Delta a_p$ est supérieur à une valeur seuil $A2$, $A2$ étant inférieur à $A1$,
     ◦ $\Delta b_p$ est supérieur à une valeur seuil $B1$,
     ◦ $\Delta L_p$ est inférieur à une valeur seuil $L1$.

3. Procédé selon la revendication 1 ou 2, dans lequel le seuil $A1$ est déterminé en utilisant une base de données de référence comprenant des mesures de colorimétrie réalisées sur une pluralité de premiers échantillons (12) en matériau polymère, en particulier à fibres de renfort, ayant été soumis à une température déterminée durant une période de temps déterminée.

4. Procédé selon la revendication 3, dans lequel les seuils $A2$, $B1$, $L1$ sont déterminés en utilisant lesdites mesures de colorimétrie stockées dans la base données de référence.

5. Procédé selon la revendication 3 ou 4, dans lequel l'établissement de la base de données comprend les étapes suivantes :

   - pour chaque premier échantillon (12), obtenir la valeur a' du paramètre $a$ de l'espace colorimétrique CIELAB, à partir d'au moins une mesure de colorimétrie,
   - pour chaque premier échantillon (12), calculer $\Delta a' = a' - a'_{ref}$, où :
   ◦ $a'_{ref}$ correspond à la valeur du paramètre a de l'espace colorimétrique CIELAB, cette valeur ayant été obtenue sur un second échantillon (14) en matériau polymère, en particulier à fibres de renfort, ayant le même temps d'existence que ledit premier échantillon (12) considéré et ayant été maintenu à une température comprise dans une plage de températures, telle que celle de préservation de l'intégrité mécanique du second échantillon (14) ou bien telle qu'une plage entre 0 et 40 °C pouvant prendre en compte en outre l'exposition aux rayonnements dans le domaine ultraviolet,
   - effectuer un test de détermination des propriétés mécaniques de chacun des premiers échantillons (12),
   - déterminer le seuil $A1$ à partir d'une comparaison des données issues des tests effectués à l'étape précédente et des valeurs $\Delta a'$ contenues en base de données.

6. Procédé selon la revendication 5, dans lequel l'établissement de la base de données comprend également les étapes suivantes :

- pour chaque premier échantillon (12), obtenir les valeurs $L'$ et $b'$ des paramètres respectifs $L$ et $b$ de l'espace colorimétrique CIELAB, à partir d'au moins une mesure de colorimétrie,
- pour chaque premier échantillon, calculer $\Delta b' = b' - b'_{ref}$ et $\Delta L' = L' - L'_{ref}$, où :
  ◦ $b'_{ref}$ et $L'_{ref}$ correspondent respectivement aux valeurs des paramètres $b$ et $L$ de l'espace colorimétrique CIELAB, ces valeurs ayant été obtenues sur un second échantillon (14) en matériau polymère, en particulier à fibres de renfort, ayant le même temps d'existence que ledit premier échantillon (12) considéré et ayant été maintenu à une température comprise dans une plage de températures, telle que celle de préservation de l'intégrité mécanique du second échantillon (14) ou bien telle qu'une plage entre 0 et 40 °C pouvant prendre en compte en outre l'exposition aux rayonnements dans le domaine ultraviolet,
- déterminer les seuils $A2$, $B1$ et $L1$ à partir d'une comparaison des données issues desdits tests et des valeurs $\Delta a'$, $\Delta b'$ et $\Delta L'$ contenues en base de données.

7. Procédé selon la revendication 5 ou 6, dans lequel ledit test comprend au moins une étape de test mécanique de la pièce, par exemple en traction et/ou compression.

8. Procédé selon l'une des revendications précédentes, comprenant en outre les étapes suivantes :

- calculer la différence de couleur $\Delta E_p$ entre les mesures de colorimétrie obtenues aux étapes a) et b),
- à partir de la valeur $\Delta E_p$ obtenue à l'étape précédente, déterminer la période de temps durant laquelle ladite zone à contrôler de la pièce (10) a été soumise à une température d'échauffement déterminée et déterminer ladite température d'échauffement, en utilisant une base de donnée de référence comprenant des valeurs $\Delta E'$ de différence de couleurs obtenues à partir d'une pluralité d'échantillons (12) en matériau polymère ayant été soumis à une température déterminée durant une période de temps déterminée.

9. Procédé selon l'une des revendications précédentes, **caractérisé en ce que** chaque valeur considérée des paramètres $L$, $a$, $b$ de l'espace colorimétrique CIELAB est obtenue en effectuant la moyenne d'au moins cinq mesures successives de colorimétrie à l'endroit considéré.

10. Procédé selon l'une des revendications précédentes, dans lequel en cas d'établissement d'un risque d'échauffement, le procédé comprend en outre une étape subséquente à l'étape d) de réalisation d'une analyse physico-chimique de la zone déterminée (24) à contrôler de la pièce (10) de manière à statuer sur l'état d'endommagement thermique de ladite zone déterminée.

11. Procédé selon l'une des revendications précédentes, dans lequel le matériau polymère comprend des fibres de renfort.

12. Procédé selon l'une des revendications précédentes, dans lequel il comprend une étape de nettoyage de la surface sur laquelle une mesure de colorimétrie est destinée à être réalisée.

**Patentansprüche**

1. Verfahren zur zerstörungsfreien Prüfung der Erwärmung eines bestimmten zu prüfenden Bereichs (24) eines Bauteils (10) aus einem polymeren Material, wobei das Verfahren die folgenden Schritte umfasst:

a) Durchführen zumindest einer kolorimetrischen Messung an dem bestimmten zu prüfenden Bereich (24) und Ermitteln des Wertes $a_p$ von dem Parameter a aus dem CIELAB-Farbraum,
b) Durchführen zumindest einer kolorimetrischen Messung an einem Referenzbereich (26) des Bauteils (10) und Ermitteln des Wertes $a_{p/ref}$ von dem Parameter a aus dem CIELAB-Farbraum,
c) Berechnen von $\Delta a_p = a_p - a_{p/ref}$,
d) Feststellen eines Erwärmungsrisikos des bestimmten zu prüfenden Bereichs (24), wenn $\Delta a_p$ größer als ein Schwellenwert $A1$ ist.

2. Verfahren nach Anspruch 1, ferner umfassend die folgenden Schritte:

- Ermitteln des Wertes $b_p$ von dem Parameter b und des Wertes $L_p$ von dem Parameter $L$ der zumindest einen in Schritt a) durchgeführten kolorimetrischen Messung,
- Ermitteln des Wertes $b_{p/ref}$ von dem Parameter b und des Wertes $L_{p/ref}$ von dem Parameter $L$ der zumindest einen in Schritt b) durchgeführten kolorimetrischen Messung,
- Berechnen von $\Delta b_p = b_p - b_{p/ref}$ und Berechnen von $\Delta L_p = L_p - L_{p/ref}$,
- Feststellen eines Erwärmungsrisikos des bestimmten zu prüfenden Bereichs (24), wenn alle folgenden Bedingungen erfüllt sind :

○ $\Delta a_p$ ist größer als ein Schwellenwert $A2$, wobei $A2$ kleiner als $A1$ ist,
○ $\Delta b_p$ ist größer als ein Schwellenwert $B1$,
○ $\Delta L_p$ ist kleiner als ein Schwellenwert $L1$.

**3.** Verfahren nach Anspruch 1 oder 2, wobei der Schwellenwert $A1$ unter Verwendung einer Referenzdatenbank bestimmt wird, die kolorimetrische Messungen umfasst, die an einer Vielzahl von ersten Proben (12) aus polymerem Material, insbesondere mit Verstärkungsfasern, durchgeführt wurden, nachdem sie für eine bestimmte Zeitspanne einer bestimmten Temperatur ausgesetzt wurden.

**4.** Verfahren nach Anspruch 3, wobei die Schwellenwerte $A2, B1, L1$ unter Verwendung der in der Referenzdatenbank gespeicherten kolorimetrischen Messungen bestimmt werden.

**5.** Verfahren nach Anspruch 3 oder 4, wobei der Aufbau der Datenbank die folgenden Schritte umfasst:

- für jede erste Probe (12) Ermitteln des Wert $a'$ von dem Parameter $a$ aus dem CIELAB-Farbraum ausgehend von zumindest einer kolorimetrischen Messung,
- für jede erste Probe (12) Berechnen von $\Delta a' = a' - a'_{ref}$, worin:
○ $a'_{ref}$ dem Wert des Parameters $a$ aus dem CIELAB-Farbraum entspricht, wobei dieser Wert an einer zweiten Probe (14) aus polymerem Material, insbesondere mit Verstärkungsfasern, erhalten wurde, die die gleiche Lebensdauer wie die betrachtete erste Probe (12) aufweist und bei einer Temperatur innerhalb eines Temperaturbereichs gehalten wurde, wie etwa dem zur Erhaltung der mechanischen Unversehrtheit der zweiten Probe (14) oder wie etwa einem Temperaturbereich zwischen 0 und 40°C, der auch die Einwirkung von Strahlungen im ultravioletten Bereich berücksichtigen kann,
- Durchführen eines Tests zum Bestimmen der mechanischen Eigenschaften jeder der ersten Proben (12),
- Bestimmen des Schwellenwerts $A1$ ausgehend von einem Vergleich der Daten aus den im vorherigen Schritt durchgeführten Tests und der in der Datenbank enthaltenen Werte $\Delta a'$.

**6.** Verfahren nach Anspruch 5, wobei der Aufbau der Datenbank ferner die folgenden Schritte umfasst:

- für jede erste Probe (12) Ermitteln der Werte $L'$ und $b'$ der jeweiligen Parameter $L$ und $b$ aus dem CIELAB-Farbraum ausgehend von zumindest einer kolorimetrischen Messung,
- für jede erste Probe Berechnen von $\Delta b' = b' -$

$b'_{ref}$ und $\Delta L' = L' - L'_{ref}$, worin:
○ $b'_{ref}$ und $L'_{ref}$ jeweils den Werten der Parameter $b$ und $L$ aus dem CIELAB-Farbraum entsprechen, wobei diese Werte an einer zweiten Probe (14) aus polymerem Material, insbesondere mit Verstärkungsfasern, erhalten wurden, die die gleiche Lebensdauer wie die betrachtete erste Probe (12) aufweist und bei einer Temperatur innerhalb eines Temperaturbereichs gehalten wurde, wie etwa dem zur Erhaltung der mechanischen Unversehrtheit der zweiten Probe (14) oder wie etwa einem Temperaturbereich zwischen 0 und 40 °C, der auch die Einwirkung von Strahlungen im ultravioletten Bereich berücksichtigen kann,
- Bestimmen der Schwellenwerte $A2, B1$ und $L1$ ausgehend von einem Vergleich der Daten aus den genannten Tests und der in der Datenbank enthaltenen Werte $\Delta a, \Delta b'$ und $\Delta L'$.

**7.** Verfahren nach Anspruch 5 oder 6, wobei der Test zumindest einen Schritt des mechanischen Testens des Bauteils, zum Beispiel auf Zug und/oder Druck, umfasst.

**8.** Verfahren nach einem der vorhergehenden Ansprüche, ferner umfassend die folgenden Schritte:

- Berechnen der Farbdifferenz $\Delta Ep$ zwischen den in den Schritten a) und b) erhaltenen kolorimetrischen Messungen,
- ausgehend von dem im vorhergehenden Schritt erhaltenen Wert $\Delta Ep$ Bestimmen der Zeitspanne, während der der zu prüfende Bereich des Bauteils (10) einer bestimmten Erwärmungstemperatur ausgesetzt wurde, und Bestimmen der Erwärmungstemperatur unter Verwendung einer Referenzdatenbank, die Werte $\Delta E'$ der Farbdifferenz umfasst, die von einer Vielzahl von Proben (12) aus polymerem Material erhalten wurden, die für eine bestimmte Zeitspanne einer bestimmten Temperatur ausgesetzt wurden.

**9.** Verfahren nach einem der vorhergehenden Ansprüche, **dadurch gekennzeichnet, dass** jeder betrachtete Wert der Parameter $L, a, b$ aus dem CIELAB-Farbraum durch Mittelung von zumindest fünf aufeinanderfolgenden kolorimetrischen Messungen an der betrachteten Stelle ermittelt wird.

**10.** Verfahren nach einem der vorhergehenden Ansprüche, wobei das Verfahren im Falle der Feststellung eines Erwärmungsrisikos ferner einen auf den Schritt d) folgenden Schritt der Durchführung einer physikalisch-chemischen Analyse des bestimmten zu prüfenden Bereichs (24) des Bauteils (10) umfasst, um über den Zustand der thermischen Schädigung des bestimmten Bereichs zu entscheiden.

11. Verfahren nach einem der vorhergehenden Ansprüche,

   wobei das polymere Material Verstärkungsfasern enthält.

12. Verfahren nach einem der vorhergehenden Ansprüche,

   wobei es einen Schritt des Reinigens der Oberfläche, auf der eine kolorimetrische Messung durchgeführt werden soll, umfasst.

**Claims**

1. A method for non-destructive testing of the heating of a specific zone (24) to be tested of a part (10) made of polymer material, the method including the following steps:

   a) taking at least one colorimetry measurement on said determined zone (24) to be tested and obtaining the value $a_p$ of the parameter a of the colorimetric space CIELAB,

   b) taking at least one colorimetry measurement on a reference zone (26) of said part (10) and obtaining the value $a_{p/ref}$ of the parameter a of the colorimetric space CIELAB,

   c) calculating $\Delta a_p = a_p - a_{p/ref}$,

   d) establishing a risk of heating of said predetermined zone (24) to be tested if $\Delta a_p$ is higher than a threshold value Al.

2. The method according to claim 1, which further comprises the following steps:

   - obtaining the value $b_p$ of the parameter b and the value $L_p$ of the parameter $L$ of said at least one colorimetry measurement performed in step a),

   - obtaining the value $b_{p/ref}$ of the parameter $b$ and the value $L_{p/ref}$ of the parameter $L$ of said at least one colorimetry measurement performed in step b),

   - calculating $\Delta b_p = b_p - b_{p/ref}$ and calculating $\Delta L_p = L_p - L_{p/ref}$,

   - establishing a risk of heating of said predetermined zone (24) to be tested if all the following conditions are verified:

   $\Delta a_p$ is greater than a threshold value $A2$, $A2$ being less than $Al$,

   $\Delta b_p$ is greater than a threshold value $B1$,

   $\Delta L_p$ is less than a threshold value $L1$.

3. A method according to claim 1 or 2, wherein the threshold $Al$ is predetermined using a reference database comprising colorimetry measurements performed on a plurality of first samples (12) of polymer material, in particular reinforcing fibers, having been subjected to a predetermined temperature during a predetermined period of time.

4. A method according to claim 3, wherein the thresholds $A2, B1, L1$ are predetermined using said colorimetry measurements stored in the reference database.

5. A method according to claim 3 or 4, wherein forming the database comprises the following steps:

   - for each first sample (12), obtaining the value a' of the parameter a ' of the colorimetric space CIELAB, from at least one colorimetry measurement,

   - for each first sample (12), calculating $\Delta a' = a' - a'_{ref}$, where:

   ○ $a'_{ref}$ corresponds to the value of the parameter a of the colorimetric space CIELAB, this value having been obtained on a second sample (14) made of polymer material, in particular reinforcing fibers, having the same existence time as said first sample (12) considered and having been maintained at a temperature within a temperature range, such as that of preserving the mechanical integrity of the second sample (14) or such that a range between 0 and 40°C may also take into account exposure to radiation in the ultraviolet range,

   - taking a test to determine the mechanical properties of each of the first samples (12),

   - determining the $Al$ threshold *from* a comparison of the data from the tests performed in the previous step and the values $\Delta a'$ contained in the database.

6. A method according to claim 5, wherein forming the database also comprises the following steps:

   - for each first sample (12), obtaining the values $L'$ and b' of the respective parameters $L$ and b ' of the colorimetric space CIELAB, from at least one colorimetry measurement,

   - for each first sample, calculating $\Delta b' = b' - b'_{ref}$ and $\Delta L' = L' - L'_{ref}$, where:

   ○ $b'_{ref}$ and $L'_{ref}$ respectively correspond to the values of the parameters b and $L$ of the colorimetric space CIELAB, these values having been obtained on a second sample (14) made of polymer material, in particular reinforcing fibers, having the same existence time as said first sample (12) considered and having been maintained at a temperature within a temperature range, such as that of preserving the mechanical integrity of the second sample (14) or such that a range between 0 and 40°C may also take into account exposure to radiation in the ultraviolet range,

- determining the thresholds $A2$, $B1$ and $L1$ from a comparison of the data from said tests and the values $\Delta a'$, $\Delta b'$ and $\Delta L'$ contained in the database.

7. A method according to claim 5 or 6, wherein said test comprises at least one step of mechanically testing the part, for example in tension and/or compression.

8. A method according to one of the preceding claims, further comprising the following steps:

- calculating the color difference $\Delta E_p$ between the colorimetry measurements obtained in steps a) and b),
- from the value $\Delta_{Ep}$ obtained in the previous step, determining the period of time during which said zone to be tested of the part (10) has been subjected to a predetermined heating temperature and determining said heating temperature, using a reference database comprising color difference values $\Delta E'$ obtained from a plurality of samples (12) of polymer material having been subjected to a predetermined temperature during a predetermined period of time.

9. A method according to one of the preceding claims, **characterized in that** each considered value of the parameters $L$, $a$, $b$ of the calorimetric space CIELAB is obtained by averaging at least five successive colorimetry measurements at the considered location.

10. A method according to one of the preceding claims, wherein, in the event that a risk of heating is established, the method further comprises a step subsequent to step d) of carrying out a physico-chemical analysis of the predetermined zone (24) to be tested of the part (10) in order to decide on the state of thermal damage of said predetermined zone.

11. A method according to one of the preceding claims, wherein the polymer material comprises reinforcing fibers.

12. A method according to one of the preceding claims, which comprises a step of cleaning the surface on which a colorimetry measurement is to be performed.

**Fig. 1**

**Fig. 2**

Fig. 3

Fig. 4

Fig. 5

Fig. 6

La pièce est utilisable

$\Delta Lp < L_1$
et
$\Delta ap > A_2,$
avec $A_2 < A_1$
et
$\Delta bp > B_1$

non

non

oui

La pièce n'est pas utilisable

$\Delta ap > A_1$

oui

La pièce n'est pas utilisable

**Fig. 7**

Fig. 8

Fig. 9

## RÉFÉRENCES CITÉES DANS LA DESCRIPTION

**Documents brevets cités dans la description**

- WO 2013050691 A